# EUROPEAN PATENT APPLICATION

(11) **EP 4 542 489 A1**
(43) Date of publication of application: **23.04.2025**
(21) Application number: 23306812.1
(22) Date of filing: 17.10.2023
(51) Int. Cl.: G06T 7/30, A61B 6/50, G06T 11/00

(54) **PROVIDING AN IMAGE REPRESENTATION OF A VASCULAR REGION**

(71) Applicant: Koninklijke Philips N.V., 5656 AG Eindhoven (NL)
(72) Inventor: FLORENT, Raoul, 5656 EINDHOVEN (NL); VIK, Torbjoern, 5656 EINDHOVEN (NL); HENDRIKS, Bernardus, 5656 EINDHOVEN (NL); OLIVAN-BESCOS, Javier, 5656 EINDHOVEN (NL)
(74) Representative: Philips Intellectual Property & Standards

(57) **Abstract**

A system for providing an image representation of a vascular region, is provided. The system comprises one or more processors configured to receive a volumetric angiographic image (120) of the vascular region; receive one or more projected angiographic images (130) of the vascular region; and receive a live sequence of projected fluoroscopic images (140) of the vascular region. The live sequence of projected fluoroscopic images includes at least a portion of an interventional device (150). The one or more processors calculate three-dimensional positions of the at least a portion of the interventional device in the volumetric angiographic image (120) corresponding to two-dimensional positions of the at least a portion of the interventional device in the live sequence of projected fluoroscopic images (140), based on a first registration operation (160) wherein the volumetric angiographic image (120) is registered to the one or more projected angiographic images (130), and a second registration operation (170) wherein the live sequence of projected fluoroscopic images (140) is registered to the one or more projected angiographic images (130). The one or more processors also output an image representation (180) of the vascular region. The image representation (180) is determined based on the calculated three-dimensional positions of the at least a portion of the interventional device (150) in the volumetric angiographic image (120).

## Description

### TECHNICAL FIELD

The present disclosure relates to providing an image representation of a vascular region. A system, a projection X-ray imaging system, a computer-implemented method, and a computer program product, are disclosed.

### BACKGROUND

Vascular interventions are often planned, and also carried-out, using projected images, i.e. two-dimensional images.

For instance, percutaneous coronary interventional "PCI" procedures are often planned using projected angiographic images such as two-dimensional "2D" X-ray images. The projected angiographic images are acquired subsequent to the injection of a contrast agent into the vascular region, and consequently indicate the vessels in the vascular region. The projected angiographic images are used to define a planned path through the vessels to a target location where the intervention is to be performed. The subsequent vascular intervention is then carried out using a live sequence of projected fluoroscopic images. The physician uses the live sequence of projected fluoroscopic images to navigate interventional devices to the target location whilst referring back to the planned path in the projected angiographic images. The planned path in the angiographic images therefore serves as a so-called "roadmap" for interventional device navigation during the vascular intervention. Interventional devices are typically visible in the live sequence of projected fluoroscopic images due to their radio-opacity. However, vessels are poorly visible in the fluoroscopic images because the vessels have similar radiodensity values as their surrounding tissue. A contrast agent may therefore be injected into the vasculature intermittently during acquisition of the fluoroscopic images in order to provide intermittent visibility of the vessels and to thereby ensure that the roadmap is being followed.

However, contrast agent has been associated with health risks, and it is therefore desirable to reduce the amount of contrast agent that is used during interventional device navigation.

A known technique for reducing the amount of contrast agent that is injected into the vasculature during interventional device navigation is to register the live sequence of projected fluoroscopic images to the previously-acquired projected angiographic images, as disclosed in a document WO 2012/107857 A1. The document WO 2012/107857 A1 relates to a method for providing an image representation supporting positioning of an intervention device in a vessel intervention procedure. Therein, an anatomy representation of a vessel region of interest and at least one angiogram X-ray image and a live fluoroscopy X-ray image are acquired. The following steps are performed when a radio-opaque device is fixedly arranged within the vessel region of interest: (a) registering the anatomy representation to the at least one angiogram X-ray image in order to provide an anatomy-angiogram-registration; (b) processing the at least one angiogram X-ray image and the at least one live fluoroscopy X-ray image in order to identify in each of the X-ray images the radio-opaque device; (c) registering the at least one angiogram X-ray image to the at least one live fluoroscopy X-ray image based on the identified radio-opaque device in order to provide an angiogram-fluoroscopy-registration; and (d) combining the anatomy-angiogram-registration and the angiogram-fluoroscopy-registration in order to provide an anatomy-fluoroscopy-registration. Finally, an image representation resulting from the anatomy-fluoroscopy-registration showing an overlay of live images with the anatomy representation may be output thereby helping a surgeon to accurately position for example a synthetic aortic valve within an aortic root of a heart.

Despite the benefits of the technique described in the document WO 2012/107857 A1, there remain challenges to navigating interventional devices in the vasculature.

### SUMMARY

According to one aspect of the present disclosure, a system for providing an image representation of a vascular region, is provided. The system comprises one or more processors configured to:
receive first image data representing a volumetric angiographic image of the vascular region;
receive second image data representing one or more projected angiographic images of the vascular region;
receive third image data representing a live sequence of projected fluoroscopic images of the vascular region, the live sequence of projected fluoroscopic images including at least a portion of an interventional device;
calculate three-dimensional positions of the at least a portion of the interventional device in the volumetric angiographic image corresponding to two-dimensional positions of the at least a portion of the interventional device in the live sequence of projected fluoroscopic images, based on a first registration operation wherein the volumetric angiographic image is registered to the one or more projected angiographic images, and a second registration operation wherein the live sequence of projected fluoroscopic images is registered to the one or more projected angiographic images; and
output an image representation of the vascular region, wherein the image representation is determined based on the calculated three-dimensional positions of the at least a portion of the interventional device in the volumetric angiographic image.

The document WO 2012/107857 A1 mentioned above describes the use of an overlay to help a surgeon to position a synthetic aortic valve within an aortic root of a heart. The inventors have found that a drawback of the overlay that is described the document WO 2012/107857 A1 is that in the overlay there can be ambiguity over the vessel in which an interventional device is located.

In the system described herein, three-dimensional positions of the interventional device in the volumetric angiographic image corresponding to two-dimensional positions of the interventional device in the live sequence of projected fluoroscopic images, are calculated based on the first and second registration operations. In contrast to simply illustrating the position of the interventional device as an overlay of a fluoroscopic image onto a representation of the anatomy, the calculated three-dimensional positions of the interventional device in the volumetric angiographic image overcome ambiguity over the vessel in which the interventional device is located.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for providing an image representation of a vascular region, in accordance with some aspects of the present disclosure.
Fig. 2 is a flowchart illustrating an example of a computer-implemented method of providing an image representation of a vascular region, in accordance with some aspects of the present disclosure.
Fig. 3 is a schematic diagram illustrating an example of various elements of a method of providing an image representation of a vascular region, in accordance with some aspects of the present disclosure.
Fig. 4 is an example of an image representation 180 of a vascular region, in accordance with some aspects of the present disclosure.
Fig. 5 is an example of a volumetric angiographic image 120 including a back-projected distal end of an interventional device 150 having a three-dimensional position with a back-projection depth, D, along a depth axis 190, in accordance with some aspects of the present disclosure.
Fig. 6 is a schematic diagram illustrating a second example of various elements of a method of providing an image representation of a vascular region, in accordance with some aspects of the present disclosure.

### DETAILED DESCRIPTION

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of a system for providing an image representation of a vascular region. In some examples, the vascular region is a portion of the heart. It is, however, to be appreciated that the heart serves only as an example of a vascular region. In general, the vascular region may be any anatomical region. For instance, the vascular region may be a portion of the heart, the brain, the lung, the leg, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are performed by the one or more processors of the system disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there remain challenges to navigating interventional devices in the vasculature.

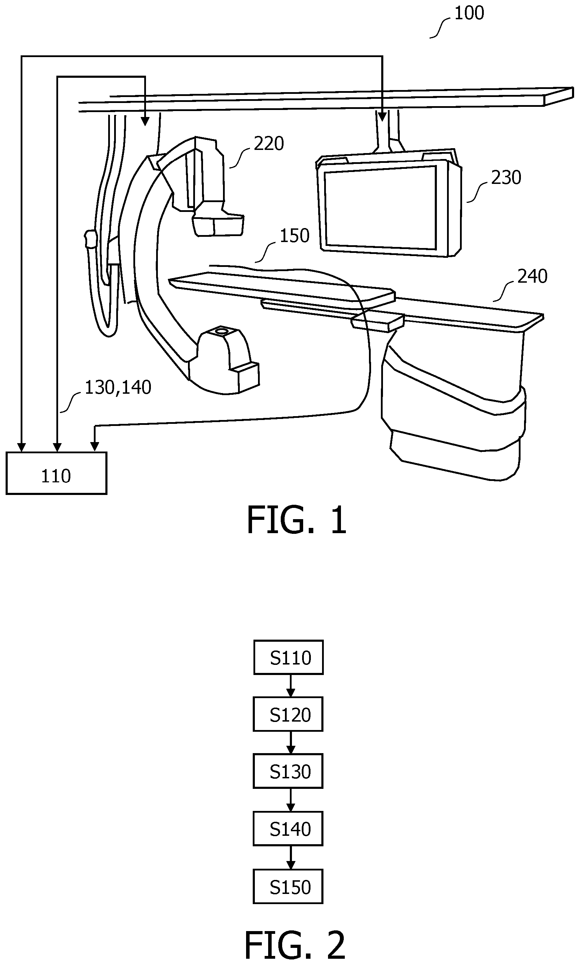

Fig. 1 is a schematic diagram illustrating an example of a system 100 for providing an image representation of a vascular region, in accordance with some aspects of the present disclosure. Fig. 2 is a flowchart illustrating an example of a computer-implemented method of providing an image representation of a vascular region, in accordance with some aspects of the present disclosure. It is noted that operations that are described as being performed by the one or more processors 110 of the system 100 illustrated in Fig. 1, may also be performed in the method illustrated in Fig. 2. Likewise, operations that are described in relation to the method described with reference to Fig. 2 may also be performed by the one or more processors 110 of the system 100 illustrated in Fig. 1. With reference to Fig. 1, and Fig. 2, the system 100 for providing an image representation of a vascular region comprises one or more processors 110 configured to:
receive S110 first image data representing a volumetric angiographic image 120 of the vascular region;
receive S120 second image data representing one or more projected angiographic images 130 of the vascular region;
receive S130 third image data representing a live sequence of projected fluoroscopic images 140 of the vascular region, the live sequence of projected fluoroscopic images including at least a portion of an interventional device 150;
calculate S140 three-dimensional positions of the at least a portion of the interventional device 150 in the volumetric angiographic image 120 corresponding to two-dimensional positions of the at least a portion of the interventional device 150 in the live sequence of projected fluoroscopic images 140, based on a first registration operation 160 wherein the volumetric angiographic image 120 is registered to the one or more projected angiographic images 130, and a second registration operation 170 wherein the live sequence of projected fluoroscopic images 140 is registered to the one or more projected angiographic images 130; and
output S150 an image representation 180 of the vascular region, wherein the image representation 180 is determined based on the calculated three-dimensional positions of the at least a portion of the interventional device 150 in the volumetric angiographic image 120.

In the above system, three-dimensional positions of the interventional device in the volumetric angiographic image corresponding to two-dimensional positions of the interventional device in the live sequence of projected fluoroscopic images, are calculated based on the first and second registration operations. In contrast to simply illustrating the position of the interventional device as an overlay of a fluoroscopic image onto a representation of the anatomy, the calculated three-dimensional positions of the interventional device in the volumetric angiographic image overcome ambiguity over the vessel in which the interventional device is located.

The operations performed by the system 100 are described in more detail below.

In the operation S110 first image data is received. The first image data represents a volumetric angiographic image 120 of the vascular region.

The volumetric angiographic image 120 that is received in the operation S110 may be generated by various types of volumetric imaging systems. These include computed tomography "CT" imaging systems, magnetic resonance imaging "MRI" systems, and ultrasound imaging systems. The CT imaging system may be a spectral CT imaging system. Spectral CT imaging systems generate X-ray attenuation data representing X-ray attenuation within multiple different energy intervals. The X-ray attenuation data generated by a spectral CT imaging system may be processed, e.g. using various material decomposition algorithms, in order to distinguish between media that have similar X-ray attenuation values when measured within a single energy interval, and which would be indistinguishable in X-ray attenuation data obtained using a conventional CT imaging system. Thus, X-ray attenuation data generated by spectral CT imaging systems may be processed to provide volumetric angiographic images with improved specificity to materials such as contrast agent, tissue, bone, and so forth.

With continued reference to the operation S110, in one example, the volumetric angiographic image 120 are generated subsequent to the injection of a contrast agent into the vasculature of a subject. Thus, in this example, the volumetric angiographic image 120 represents a distribution of a contrast agent in the vascular region. The contrast agent serves to improve the contrast between blood and surrounding tissue in the volumetric angiographic image 120. In this example, the volumetric angiographic image 120 may be referred-to as contrast-enhanced volumetric angiographic image. Contrast-enhanced volumetric angiographic images may be generated by a CT imaging system, or by an MRI system. MRI volumetric angiographic images may alternatively be generated in the absence of a contrast agent, and are often referred-to as non-contrast-enhanced MR angiography "NC-MRA" images.

With continued reference to the operation S110, the volumetric angiographic image 120 may be received from various sources in the operation S 110, including from a medical imaging system such as one of the imaging systems described above. Alternatively, the volumetric angiographic image 120 may be received from another source, such as from a computer readable storage medium, or from the internet, or from the Cloud, and so forth. In general, the volumetric angiographic image 120 may be received via any form of data communication, including wired and wireless communication. By way of some examples, when wired communication is used, the communication may take place via an electrical or optical cable, and when wireless communication is used, the communication may for example take place via RF or infrared signals.

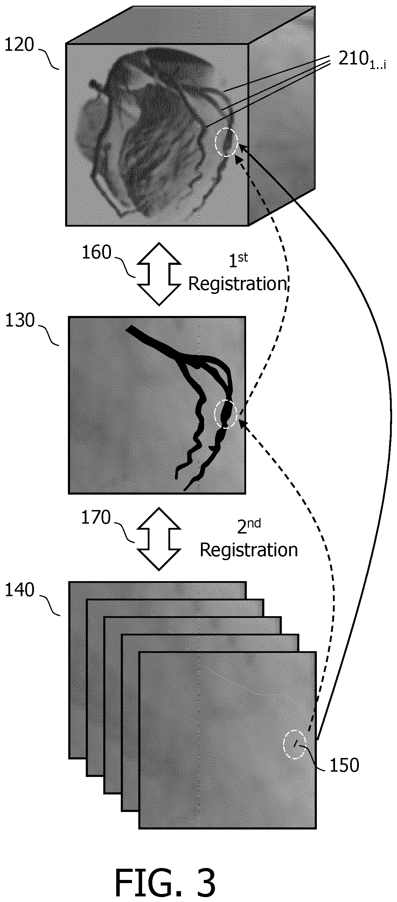

An example of a volumetric angiographic image 120 that is received in the operation S110 is illustrated in Fig. 3, which is a schematic diagram illustrating an example of various elements of a method of providing an image representation of a vascular region, in accordance with some aspects of the present disclosure. With reference to the volumetric angiographic image 120 illustrated in the upper portion of Fig. 3, in this example the volumetric angiographic image 120 represents the heart and the volumetric angiographic image 120 includes a plurality of vessels 210_{1..i}. The vessels 210_{1..i.} may include various types of vessels, such as arteries, veins, and so forth. In one example, the vessels 210_{1..i.} include one or more coronary arteries.

Referring now to the operation S120 mentioned above with reference to Fig. 1 and Fig. 2; in this operation, second image data is received. The second image data represents one or more projected angiographic images 130 of the vascular region.

The one or more projected angiographic images 130 that are received in the operation S120 may be generated by various types of projection X-ray imaging systems. The projection X-ray imaging system may be a spectral X-ray projection imaging system. Spectral X-ray projection imaging systems generate X-ray attenuation data representing X-ray attenuation within multiple different energy intervals. The X-ray attenuation data generated by a spectral X-ray projection imaging system may be processed, e.g. using various material decomposition algorithms, in order to distinguish between media that have similar X-ray attenuation values when measured within a single energy interval, and which would be indistinguishable in X-ray attenuation data obtained using a conventional projection X-ray imaging system. Thus, X-ray attenuation data generated by a spectral X-ray imaging system, may be processed to provide projected angiographic images with improved specificity to materials such as contrast agent, tissue, bone, and so forth.

With continued reference to the operation S120, in one example, the one or more projected angiographic images 130 that are received in the operation S120 are generated subsequent to the injection of a contrast agent into the vasculature of a subject. Thus, in this example, the projected angiographic image(s) 130 represent a distribution of a contrast agent in the vascular region. The contrast agent serves to improve the contrast between blood and surrounding tissue in the one or more projected angiographic images 130. In this example, the one or more projected angiographic images 130 may be referred-to as contrast-enhanced projected angiographic images 130.

In one example, the one or more projected angiographic images 130 that are received in the operation S120 are generated during the performance of a medical procedure on the anatomical region. In this example, the one or more projected angiographic images 130 may be referred-to as a intra-procedural projected angiographic images 130. In one example, the one or more projected angiographic images 130 that are received in the operation S120 represent a temporal sequence. The images in the temporal sequence may be generated periodically, or at irregular intervals. In a related example, the second angiographic image data represents a sequence of projected angiographic images 130 of the vascular region, and the sequence of projected angiographic images 130 is acquired prior to the live sequence of projected fluoroscopic images 140 that is acquired in the operation S130.

With continued reference to the operation S120, the one or more projected angiographic images 130 may be received from various sources in the operation S120, including from a medical imaging system such as one of the imaging systems described above. Alternatively, the one or more projected angiographic images 130 may be received from another source, such as from a computer readable storage medium, or from the internet, or from the Cloud, and so forth. In general, the one or more projected angiographic images 130 may be received via any form of data communication.

An example of a projected angiographic image 130 that is received in the operation S120 is illustrated in the central portion of Fig. 3. The projected angiographic image 130 illustrated in Fig. 3 represents the same heart that is illustrated in the volumetric angiographic image 120 in the upper portion of Fig. 3. It may be noted that fewer vessels are represented in the projected angiographic image 130 than in the volumetric angiographic image 120. In general, some, or all, of the vessels represented in the volumetric angiographic image 120 may be represented in the projected angiographic image 130. The contrast agent that is injected into the vasculature in order to obtain the projected angiographic image 130 may be injected intra-arterially, for example it may be injected into a cardiac chamber, or alternatively it may be injected intra-venously. In the former case, arteries that are distal to the point of injection may be imaged. In the latter case, the contrast agent typically flows though the full venous and arterial system, and consequently all vessels may be imaged. The example projected angiographic image 130 illustrated in Fig. 3 corresponds to the former case wherein the contrast agent is injected in to the ostium of the left coronary tree, and consequently the vessels that are represented in the projected angiographic image 130 are limited to the arteries in the left coronary tree that are downstream of the point of injection.

Referring now to the operation S130 mentioned above with reference to Fig. 1 and Fig. 2; in this operation, third image data is received. The third image data represents a live sequence of projected fluoroscopic images 140 of the vascular region. The live sequence of projected fluoroscopic images includes at least a portion of an interventional device 150.

The live sequence of projected fluoroscopic images 140 that are received in the operation S130 may be generated by various types of projection X-ray imaging systems. The projection X-ray imaging system may be a spectral X-ray projection imaging system. The live sequence of projected fluoroscopic images 140 may be generated by the same type of projection X-ray imaging system that generates the one or more projected angiographic images 130. An orientation of the projection X-ray imaging system that is used to acquire the live sequence of projected fluoroscopic images 140 may be similar, or the same, with respect to the vascular region, as the orientation of the projection X-ray imaging system that is used to acquire the one or more projected angiographic images 130. Consequently, the live sequence of projected fluoroscopic images 140 and the one or more projected angiographic images 130 represent a similar perspective of the vascular region.

With continued reference to the operation S130, the live sequence of projected fluoroscopic images includes at least a portion of an interventional device 150. In general, the interventional device may be any type of intravascular device. For example, the interventional device may be a guidewire, a catheter, an intravascular imaging device such as an intravascular ultrasound imaging "IVUS" imaging device, or an optical coherence tomography "OCT" imaging device. The portion of the interventional device may be a tip, i.e. a distal end, or another portion of the interventional device. The portion of the interventional device may be formed from a radiopaque material in order to provide visibility of the interventional device in the fluoroscopic images. For instance, a tip of a catheter may be formed from a radiopaque material, or the interventional device may include a radiopaque marker.

With continued reference to the operation S130, the images in the live sequence of projected fluoroscopic images 140 are generated instantaneously before being received by the one or more processors 110. In other words, they are both generated and received substantially in real-time. By contrast, the one or more projected angiographic images 130 that are received in the operation S120 may have been generated some seconds, minutes, hours, or even days, or a longer period, before the generation of the images in the live sequence of projected fluoroscopic images 140.

With continued reference to the operation S130, the live sequence of projected fluoroscopic images 140 may be received from a medical imaging system such as one of the imaging systems described above. In general, the one or more projected angiographic images 130 may be received via any form of data communication.

An example of a live sequence of projected fluoroscopic images 140 that is received in the operation S130 is illustrated in the lower portion of Fig. 3. In this example, the live sequence of projected fluoroscopic images 140 is acquired in the absence of a contrast agent in the vascular region. The live sequence of projected fluoroscopic images 140 includes at least a portion of an interventional device in the form of a radiopaque tip of a catheter. As may be seen in the live sequence of projected fluoroscopic images 140 illustrated in Fig. 3, the vasculature is poorly represented in these images whereas the radiopaque tip of the catheter 150 is clearly visible.

Referring now to the operation S140 mentioned above with reference to Fig. 1 and Fig. 2; in this operation, three-dimensional positions of the at least a portion of the interventional device 150 in the volumetric angiographic image 120 corresponding to two-dimensional positions of the at least a portion of the interventional device 150 in the live sequence of projected fluoroscopic images 140, are calculated. The three dimensional positions of the at least a portion of the interventional device 150 are calculated based on a first registration operation 160 wherein the volumetric angiographic image 120 is registered to the one or more projected angiographic images 130, and a second registration operation 170 wherein the live sequence of projected fluoroscopic images 140 is registered to the one or more projected angiographic images 130.

An example of the operation S140 is illustrated in Fig. 3. The first registration operation 160 and the second registration operation 170 are illustrated via the solid arrows labelled "1^{st} registration" and "2^{nd} registration" respectively. Together, the first registration operation 160 and the second registration operation 170 define a transform comprising a back-projection of the two-dimensional positions of the at least a portion of the interventional device 150 onto the corresponding three-dimensional positions of the at least a portion of the interventional device 150 in the volumetric angiographic image 120. The individual steps of this back-projection are illustrated via the individual thin dashed arrows in Fig. 3. Their combined effect is illustrated via the thin solid arrow which connects the position of the tip of the catheter 150 in the projected fluoroscopic images 140 with the position of the tip of the catheter 150 in the volumetric angiographic image 120.

The operation S140 may be said to register the two-dimensional positions of the at least a portion of the interventional device 150 in the live sequence of projected fluoroscopic images 140 to corresponding three-dimensional positions of the at least a portion of the interventional device 150 in the volumetric angiographic image 120, via the one or more projected angiographic images 130. The one or more projected angiographic images 130 facilitate this registration because i) one or more of the vessels that are represented in the volumetric angiographic image 120 are also represented in the projected angiographic image(s) 130, and because ii) the live sequence of projected fluoroscopic images 140 and the one or more projected angiographic images 130 both represent a similar perspective of the vascular region. Moreover, if the interventional device is an intravascular device, the portion of the interventional device, which is visible in the live sequence of projected fluoroscopic images 140, is also constrained to a position within a vessel in both the volumetric angiographic image 120 and in the projected angiographic image(s) 130.

Registering the two-dimensional positions of the at least a portion of the interventional device 150 in the live sequence of projected fluoroscopic images 140, to corresponding three-dimensional positions of the at least a portion of the interventional device 150 in the volumetric angiographic image 120, via the one or more projected angiographic images 130, obviates the difficulty of directly registering the two-dimensional positions of the at least a portion of the interventional device 150 in the live sequence of projected fluoroscopic images 140 to corresponding three-dimensional positions of the at least a portion of the interventional device 150 in the volumetric angiographic image 120. As may be appreciated from Fig. 3, such a direct registration would be difficult to perform, and also inaccurate, owing to the poor visibility of the vasculature in the live sequence of projected fluoroscopic images 140. This would severely hamper, if not preclude, a direct registration of the vasculature in the live sequence of projected fluoroscopic images 140 to the vasculature in the volumetric angiographic image 120.

The first registration operation 160 and the second registration operation 170 that are performed in the operation S140 may be performed using techniques such as feature-based image registration techniques, intensity-based image registration techniques, neural networks, and so forth. An example of a technique that may be used to perform the first registration operation 160 is disclosed in a document by Markelj, P. et al., "A review of 3D/2D registration methods for image-guided interventions", Medical Image Analysis, 16, 2012, pages 642-661. An example of a technique that may be used to perform the second registration operation 170 is disclosed in the document WO 2008/104921 A2. In some examples, rigid registrations may be used in the first registration operation 160 and/or in the second registration operation 170. In other examples, elastic registrations may be used in the first registration operation 160 and/or in the second registration operation 170, as described in the examples below.

In one example a single volumetric angiographic image 120 is received in the operation S110. The single volumetric angiographic image 120 may represent a time in the cardiac cycle, such as at an intermediate cardiac phase between systole and diastole. In this example, the first registration operation 160, i.e. wherein the volumetric angiographic image 120 is registered to the one or more projected angiographic images 130, may be performed using a single projected angiographic image 130 of the vascular region, or it may be performed using multiple projected angiographic image 130 of the vascular region.

If a single projected angiographic image 130 is used and the projected angiographic image 130 represents a time in the cardiac cycle that is at the same cardiac phase as the volumetric angiographic image 120, the first registration operation 160 may be a rigid registration. Performing the first registration operation 160 using a rigid registration reduces processing complexity as compared to a non-rigid, i.e. elastic registration. The first registration operation 160 may alternatively be performed using an elastic registration, and which may result in an improved registration, at the expense of increased processing complexity. The second registration operation 170 may be a rigid registration, or it may be an elastic registration. For instance, if it is desired to update the three-dimensional positions of the at least a portion of the interventional device 150 in the volumetric angiographic image 120 once per cardiac cycle, and at the same cardiac phase, the second registration operation 170 may be a rigid registration. The rigid registration may be performed by selecting for each cardiac cycle an image from the live sequence of projected fluoroscopic images 140 that corresponds to the same cardiac phase as the projected angiographic image 130, and rigidly registering the selected projected fluoroscopic 140 image to the projected angiographic image 130. The second registration operation 170 may alternatively be performed using an elastic registration, and which may result in an improved registration, at the expense of increased processing complexity. For example, using an elastic registration in the second registration operation 170 may provide a more accurate registration if the presence of the at least a portion of the interventional device in a vessel in the projected fluoroscopic images 140 deforms the vessel. Such deformation may render a rigid registration inaccurate. If it is instead desired to update the three-dimensional positions of the at least a portion of the interventional device 150 in the volumetric angiographic image 120 multiple times per cardiac cycle, the second registration operation 170 may be an elastic registration. The elastic registration takes account of the deformation of the vasculature in the live sequence of projected fluoroscopic images 140 throughout the cardiac cycle.

Alternatively, if multiple projected angiographic image 130 are available, each representing times in the cardiac cycle that are at different cardiac phases, the first registration operation 160 between each of the projected angiographic images 130 and the single volumetric angiographic image 120 may be an elastic registration. The elastic registration takes account of the deformation of the vasculature in the projected angiographic images 130 throughout the cardiac cycle. In this situation, the second registration operation 170 may be a rigid registration, or it may be an elastic registration. For instance, if it is desired to update the three-dimensional positions of the at least a portion of the interventional device 150 in the volumetric angiographic image 120 only at times in the cardiac cycle corresponding to the times of the projected angiographic images 130, the second registration operation 170 may be a rigid registration. The rigid registration may be performed by selecting for each cardiac cycle an image from the live sequence of projected fluoroscopic images 140 that corresponds to the same cardiac phase as each of the projected angiographic images 130, and rigidly registering the selected projected fluoroscopic 140 image to the projected angiographic image 130. The second registration operation 170 may alternatively be performed using an elastic registration, and which may result in an improved registration, at the expense of increased processing complexity. For example, using an elastic registration in the second registration operation 170 may provide a more accurate registration if the presence of the interventional device in a vessel in the projected fluoroscopic images 140 deforms the vessel. Such deformation may render a rigid registration inaccurate.

In another example, multiple volumetric angiographic images 120 are received in the operation S110. The multiple volumetric angiographic images 120 correspond to different times in the cardiac cycle. In this example, a rigid registration may be used in both the first registration operation 160, and in the second registration operation 170, in order to update the three-dimensional positions of the at least a portion of the interventional device 150 in the volumetric angiographic image 120 at times in the cardiac cycle for which both a projected angiographic image 130 and a volumetric angiographic image 120 exist. Alternatively, an elastic registration may be used in the first registration operation 160 and/or in the second registration operation 170 to update the three-dimensional positions of the at least a portion of the interventional device 150 in the volumetric angiographic image 120 at intermediate times in the cardiac cycle, or to account for vessel deformation as a result of the presence of the interventional device in a vessel in the projected fluoroscopic images.

In another example, if multiple volumetric angiographic images 120 are received in the operation S110, and/or if multiple projected angiographic images 130 are received in the operation S120, these images may be interpolated to provide images at intermediate times in the cardiac cycle in order to avoid the use of elastic registrations in the first registration operation 160 and in the second registration operation 170, respectively.

It is noted that the operation S140 may also include an operation of identifying the at least a portion of the interventional device 150 in the live sequence of projected fluoroscopic images 140. This operation may be performed using known segmentation techniques, or known feature detection algorithms, or using a neural network that is trained to detect the portion of the interventional device 150. The operation S140 may also include an operation of segmenting the vessels in the vascular region in the volumetric angiographic image 120 and/or in the one or more projected angiographic images 130. This operation may be performed using known segmentation techniques.

Referring now to the operation S150 mentioned above with reference to Fig. 1 and Fig. 2; in this operation, an image representation 180 of the vascular region is outputted. The image representation 180 is determined based on the calculated three-dimensional positions of the at least a portion of the interventional device 150 in the volumetric angiographic image 120.

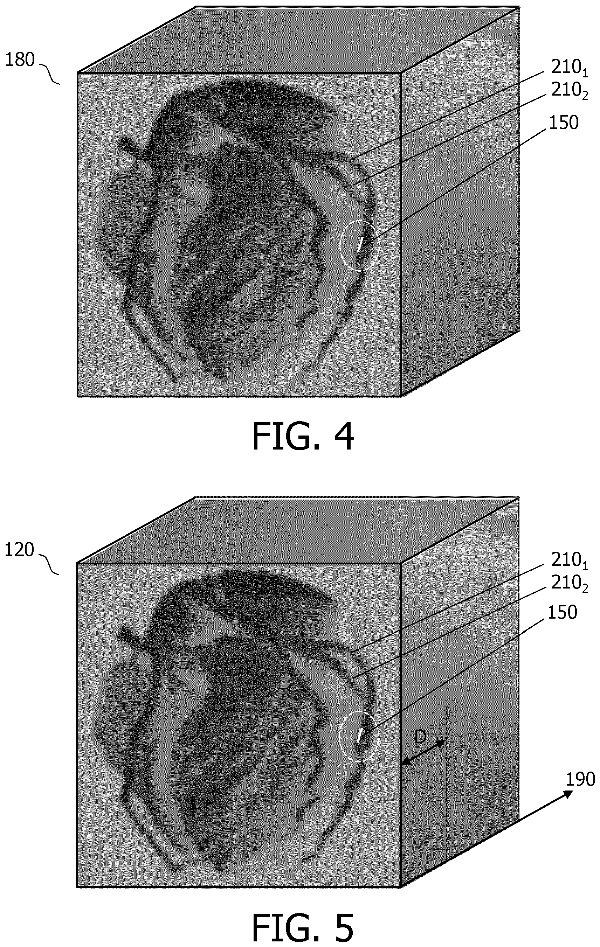

Fig. 4 is an example of an image representation 180 of a vascular region, in accordance with some aspects of the present disclosure. In the example illustrated in Fig. 4, image representation 180 comprises the at least a portion of the interventional device 150 in the calculated three-dimensional position in the volumetric angiographic image 120. The at least a portion of the interventional device 150 may be illustrated in the image representation 180 using various techniques, such as by providing a icon indicative of a portion of the interventional device, or by mapping an image of a portion of the interventional device from the live sequence of projected fluoroscopic images 140 onto the volumetric angiographic image 120.

In another example, the image representation 180 comprises a perspective view of the vascular region from the calculated three-dimensional positions of the at least a portion of the interventional device 150 in the volumetric angiographic image 120. In this example, the portion of the interventional device 150 itself may not be displayed, and rather, a perspective view from within the vascular region in the volumetric angiographic image 120, is generated. Examples of this view include a forward-looking view from a distal end of the interventional device, or a panoramic view around a distal end of the interventional device, for example. The latter view represents a view that may be provided by a virtual camera disposed at the distal end of the interventional device.

The image representation 160 may be outputted in various ways in the operation S150. For instance, the image representation 160 may be outputted to a display device, such as to the monitor 230 illustrated in Fig. 1, or to a virtual/ augmented reality display device, or to printer, or to a computer-readable storage medium, and so forth.

Referring now in more detail to the registrations that are performed in the operation S140 mentioned above with reference to Fig. 1 and Fig. 2; in this operation, the first registration operation 160 and the second registration operation 170 may be said to define a transform comprising a back-projection of the two-dimensional positions of the at least a portion of the interventional device 150 lying in a plane in the live sequence of projected fluoroscopic images 140, along a depth axis 190 onto the corresponding three-dimensional positions of the at least a portion of the interventional device 150 in the volumetric angiographic image 120, and wherein each three-dimensional position comprises a back-projection depth, D, along the depth axis 190, the back-projection depth, D, being within a vessel in the vascular region, and the depth axis being defined normally with respect to the plane of the live sequence of projected fluoroscopic images 140.

An example of a back-projection depth is illustrated in Fig. 5, which is an example of a volumetric angiographic image 120 including a back-projected distal end of an interventional device 150 having a three-dimensional position with a back-projection depth, D, along a depth axis 190, in accordance with some aspects of the present disclosure. The back-projection depth, D, to which the portion of the interventional device is projected in the volumetric angiographic image 120 is within the vessel 210₁. The back-projection depth, D, is provided by the first and second registration operations, and which result in a mapping of the two-dimensional positions of the at least a portion of the interventional device 150 in the live sequence of projected fluoroscopic images 140 to corresponding three-dimensional positions of the at least a portion of the interventional device 150 in the volumetric angiographic image 120.

The back-projection described above typically results in an accurate three-dimensional positions of the interventional device 150 in the volumetric angiographic image 120. However, an issue may arise when the vessels 210_{1..i.} in the volumetric angiographic image 120 may overlap. More specifically, when the back-projected position of the interventional device intersects with multiple vessels along the depth axis 190 in the volumetric angiographic image 120, it can be ambiguous as to which vessel the interventional device should be back-projected into. In such situations it is proposed to select one of the intersected vessels into which the interventional device 150 should be mapped. Various techniques are proposed for selecting an intersected vessels in the volumetric angiographic image 120, as described in the following examples.

In one example, for two-dimensional positions of the at least a portion of the interventional device 150 in the live sequence of projected fluoroscopic images 140 which when back-projected along the depth axis 190 intersect with multiple vessels 210₁, 210₂ in the volumetric angiographic image 120, the one or more processors 110 are configured to calculate S140 the three-dimensional position of the at least a portion of the interventional device 150 in the volumetric angiographic image 120 by back-projecting the two-dimensional position of the at least a portion of the interventional device 150 into a selected one of the intersected vessels 210₁, 210₂ to define the back-projection depth; and
wherein the selected intersected vessel 210₁, 210₂ is determined based on one or more of:
a comparison between an orientation of the at least a portion of the interventional device 150 in the live sequence of projected fluoroscopic images 140, and an orientation of the intersected vessel 210₁, 210₂ in the volumetric angiographic image 120;
a comparison between a measurement of a dimension of a feature of the vessel 210₁, 210₂ in the one or more projected angiographic images 130, and a corresponding measurement of the dimension of the feature in the intersected vessel 210₁, 210₂ in the volumetric angiographic image 120; and
a comparison between a position of an anatomical landmark in the vessel 210₁, 210₂ in the one or more projected angiographic images 130, and a corresponding position of the anatomical landmark in the intersected vessel 210₁, 210₂ in the volumetric angiographic image 120.

In the first of these examples, the orientation of the at least a portion of the interventional device 150 in the live sequence of projected fluoroscopic images 140, is compared with an orientation of the intersected vessel 210₁, 210₂ in the volumetric angiographic image 120. In this example, the selected intersected vessel in the volumetric angiographic image 120, is the intersected vessel having the closest-matching orientation to the orientation of the at least a portion of the interventional device 150 in the live sequence of projected fluoroscopic images 140. The intersected vessel having the closest-matching orientation is the most likely vessel in which the interventional device is located because the orientation of the portion of the interventional device is constrained by the orientation of the vessel in which it is located.

In the second of these examples, a measurement of a dimension of a feature of the vessel 210₁, 210₂ in the one or more projected angiographic images 130, is compared with a corresponding measurement of the dimension of the feature in the intersected vessel 210₁, 210₂ in the volumetric angiographic image 120. In this example, the selected intersected vessel in the volumetric angiographic image 120, is the intersected vessel having the closest-matching measurement to the measurement in the projected angiographic image(s) 130. In this example, the measurement may be a measurement of a width of a vessel, or a measurement of a curvature of a vessel, for example. The intersected vessel having the closest-matching measurement is the most likely vessel in which the interventional device is located because the interventional device should be mapped from a vessel in the projected angiographic image(s) 130 to the same vessel in the volumetric angiographic image 120.

In the third of these examples, a position of an anatomical landmark in the vessel 210₁, 210₂ in the one or more projected angiographic images 130, is compared with a corresponding position of the anatomical landmark in the intersected vessel 210₁, 210₂ in the volumetric angiographic image 120. In this example, the selected intersected vessel in the volumetric angiographic image 120 is the intersected vessel having the anatomical landmark in the closest-matching position to that in the projected angiographic image(s) 130. In this example, the anatomical landmark may be a bifurcation in the vessel, a calcification in the vessel, a lesion in the vessel, or a portion of a vessel abutting an occlusion in the vessel, for example. The intersected vessel having the anatomical landmark in the closest-matching position is the most likely vessel in which the interventional device is located because the interventional device should be mapped from a vessel in the projected angiographic image(s) 130 to the same vessel in the volumetric angiographic image 120.

The above examples may also be combined so as to provide a weighted likelihood of the interventional device being in an intersected vessel, the selected intersected vessel then being determined using the weighted likelihood.

In the above examples, the orientation of the at least a portion of the interventional device 150, the dimension of a feature of the vessel 210₁, 210₂, and the position of the anatomical landmark in the vessel, may be determined in the relevant images using scale-space or morphological techniques. A feature detector, or using a neural network that is trained to detect the relevant feature, may alternatively be used. One example of a neural network-based segmentation technique that may be used for this purpose is described in a document by Isensee, F., et al., "Automated Design of Deep Learning Methods for Biomedical Image Segmentation", pages 1-55, https://arxiv.org/pdf/1904.08128.pdf.

In another example, ambiguity such as that described above, is addressed by mapping the two-dimensional positions of the interventional device 150 in consecutive images in the live sequence of projected fluoroscopic images 140 to corresponding three-dimensional positions in the same vessel 210_{1..i.} in the volumetric angiographic image 120.

In this example, the one or more processors 110 are configured to calculate S140 the three-dimensional positions of the at least a portion of the interventional device 150 in the volumetric angiographic image 120 such that the two-dimensional positions of the at least a portion of the interventional device 150 in consecutive images in the live sequence of projected fluoroscopic images 140 are mapped to corresponding three-dimensional positions in the same vessel 210_{1..i.} in the volumetric angiographic image 120.

This example reduces the risk that the three-dimensional positions of the at least a portion of the interventional device 150 in the volumetric angiographic image 120 changes between vessels when multiple vessels intersect one another in the volumetric angiographic image 120. In other words, it forces a temporal coherence onto the vessel into which the portion of the interventional device 150 is located in the volumetric angiographic image 120. In this example it may be ensured that the portion of the interventional device 150 remains in the same vessel in the volumetric angiographic image 120 by constraining the position of the portion of the interventional device 150 to a path within the same vessel in the volumetric angiographic image 120. In this example it may be ensured that the portion of the interventional device 150 remains in the same vessel in the volumetric angiographic image 120 by adjusting the registration that is performed in the first registration operation 160 and/or the second registration operation 170.

In another example, ambiguity such as that described above, is addressed by labelling the vessels in the volumetric angiographic image 120 and in the projected angiographic image(s) 130 and then ensuring that the position of interventional device 150 in the live sequence of projected fluoroscopic images 140, is mapped to the same vessel 210_{1..i.} in both the projected angiographic image(s) 130 and in the volumetric angiographic image 120.

In this example, the vascular region comprises a plurality of vessels 210_{1..i}, and the one or more processors 110 are configured to label the vessels 210_{1..i.} in the volumetric angiographic image 120, and in the one or more projected angiographic images 130; and
wherein the one or more processors 110 are configured to calculate S140 the three-dimensional positions of the at least a portion of the interventional device 150 in the volumetric angiographic image 120 by:
mapping the two-dimensional positions of the at least a portion of the interventional device 150 in the live sequence of projected fluoroscopic images 140, to the corresponding three-dimensional positions of the at least a portion of the interventional device 150 in the volumetric angiographic image 120, based on the first registration operation 160, and the second registration operation 170, and via the one or more projected angiographic images 130, such that the two-dimensional positions of the at least a portion of the interventional device 150 in the vessels 210_{1..i.} in the live sequence of projected fluoroscopic images 140, are mapped to the corresponding three-dimensional positions of the at least a portion of the interventional device 150 in the same vessel 210_{1..i.} in both the one or more projected angiographic images 130 and in the volumetric angiographic image 120.

In this example, the labels are used to ensure that the interventional device is mapped to the same vessel in both the projected angiographic image(s) 130 and in the volumetric angiographic image 120. This reduces the risk that the three-dimensional positions of the at least a portion of the interventional device 150 in the volumetric angiographic image 120 changes between vessels when multiple vessels intersect one another in the volumetric angiographic image 120. This example may be useful in situations in which the portion of the interventional device 150 that is visible in the live sequence of projected fluoroscopic images 140 has a comparatively short length. In such situations there is comparatively less information on the positions of other portions of the interventional device in the volumetric angiographic image 120 into which the portion of the interventional device could be mapped, and thereby used to confirm that the interventional device is in a valid position, i.e. within a vessel. In this example, the labels may be generated using various segmentation techniques, including model-based segmentation, semantic segmentation, and so forth. With reference to the example illustrated in Fig. 3, the labels that are applied may include labels for the left anterior descending artery, "LAD", the right coronary artery "RCA", the right circumflex artery "RCX", and so forth. An example of a general segmentation technique that may be used to apply the labels is disclosed in a document by Ronneberger, O., et al., "U-Net: Convolutional Networks for Biomedical Image Segmentation", In: Navab, N., Hornegger, J., Wells, W., Frangi, A. (Eds.) Medical Image Computing and Computer-Assisted Intervention - MICCAI 2015. MICCAI 2015. Lecture Notes in Computer Science, Vol. 9351. Another example of a technique that may be used to apply the labels is disclosed in a document by Ren, P., et al., "Evaluation of a deep learning-based automated algorithm for labeling coronary arteries in computed tomography angiography images", 23 August 2022, PREPRINT (Version 1) available at Research Square, https://doi.org/10.21203/rs.3.rs-1911972/v1.

In another example, ambiguity such as that described above, is addressed by pruning, i.e. excising, the vessels in the volumetric angiographic image. In this example, the vascular region comprises a plurality of vessels 210_{1..i}, and the one or more processors 110 are configured to prune the vessels 210_{1..i.} in the volumetric angiographic image 120 such that the vessels in the volumetric angiographic image correspond to the vessels in the one or more projected angiographic images 130.

In this example, the pruning may be performed by applying an image mask to the volumetric angiographic image 120, or by applying a logical condition to the pruned vessels in the volumetric angiographic image 120 that prevents the three-dimensional positions of the at least a portion of the interventional device 150 from being located in the pruned vessels.

The pruning that is performed in this example limits the vessels in the volumetric angiographic image 120 to those that are present in the projected angiographic image(s) 130. This reduces the risk that the portion of the interventional device is mapped to the wrong vessel. In one example, the pruning is performed by automatically detecting a portion of the vasculature in which the interventional device is located, and then automatically pruning the vessels in the volumetric angiographic image 120 such that the vessels in the volumetric angiographic image correspond to the vessels in the one or more projected angiographic images 130. For instance, a trained neural network may be used to localize the position of the interventional device to a left coronary tree, or to a right coronary tree. The vessels in the volumetric angiographic image from the side in which the interventional device is not located may then be pruned from the volumetric angiographic image 120.

In another example, ambiguity such as that described above, is addressed using a three-dimensional model of the interventional device 150. In this example, the vascular region comprises a plurality of vessels 210_{1..i}, and the one or more processors 110 are configured to calculate S140 the three-dimensional positions of the at least a portion of the interventional device 150 in the volumetric angiographic image 120 by:
fitting a three-dimensional model of the interventional device 150 to the at least a portion of the interventional device in the live sequence of projected fluoroscopic images 140; and
constraining the three-dimensional positions of the at least a portion of the interventional device 150 in the volumetric angiographic image 120, using the fitted three-dimensional model, such that the three-dimensional positions of the at least a portion of the interventional device 150 lie within one of the vessels 210_{1..i.} in the volumetric angiographic image 120.

Thus, in this example, ambiguity over the vessel in which the interventional device is located is reduced by using the model to constrain the position of the interventional device such that it lies in a valid position in the volumetric angiographic image 120. In this example, the fitting, and the constraining operations may be performed using the technique disclosed in the document by Haase, C., et al., "3D ablation catheter localization using individual C-arm x-ray projections", Phys Med Biol. 2014 Nov 21; 59(22):6959-77. doi: 10.1088/0031-9155/59/22/6959. Epub 2014 Oct 28. PMID: 25350552, for instance. Alternatively, the fitting and the constraining operations in this example may be performed using the technique disclosed in the document by Haase, C., et al., "Model based 3D CS-catheter tracking from 2D X-ray projections: binary versus attenuation models", Comput Med Imaging Graph. 2014 Apr; 38(3):224-31. doi: 10.1016/j.compmedimag.2013.12.004. Epub 2013 Dec 21. PMID: 24444681.
In another example, the one or more processors 110 are configured to calculate S140 the three-dimensional positions of the at least a portion of the interventional device 150 in the volumetric angiographic image 120 by:
mapping the positions of the at least a portion of the interventional device 150 from the live sequence of projected fluoroscopic images 140 to corresponding nearest positions along a centerline of a vessel in the volumetric angiographic image 120, or to corresponding nearest positions in the vessel lying on a surface oriented normally with respect to a depth dimension of the vessel and passing half-way through the vessel along the depth dimension.

The mappings that are performed in this example may be performed by adjusting the registrations that are performed in the first registration operation S160 and/or the second registration operation S170. The mappings constrain the shape of the portion of the interventional device such that it remains within the vessel in the volumetric angiographic image 120. For instance, they help to reduce the risk that a curved portion of the interventional device is projected so as to pass through a wall of a vessel with a large radius of curvature in the volumetric angiographic image 120.

In another example, the one or more processors 110 are configured to evaluate an accuracy parameter for the image representation 180. The accuracy parameter is evaluated based on a deviation between the two-dimensional positions of the at least a portion of the interventional device 150 in the live sequence of projected fluoroscopic images 140, and the corresponding three-dimensional positions of the at least a portion of the interventional device 150 in the volumetric angiographic image 120. The value of the accuracy parameter is then outputted.

In this example, the accuracy parameter facilitates a physician to determine the extent to which the system 100 can be relied upon. The value of the accuracy parameter may be evaluated based on individual accuracy parameters that measure the accuracy of the operations described above. For instance, an accuracy parameter for the first registration operation 160 may be evaluated based on a projection of the 3D vessels onto the registered vessels in the one or more projected angiographic images 130 and a calculation of a vessel matching metric. An accuracy parameter for the second registration operation 170 may be evaluated based on a stability of the vessel characteristics (such as the angulation, radius, etc.) in the one or more projected angiographic images 130 to which the interventional device is registered. The location in which the interventional device is projected in the volumetric angiographic image 120 might be evaluated over time and a coherence of the location assessed. For instance, the interventional device cannot jump from one vessel to another unconnected vessel.

It is noted that in the examples described above, the system 100 may also include one or more of: a projection X-ray imaging system for providing the second image data and/or the third image data, such as for example the such as the C-arm-based projection X-ray imaging system 220 illustrated in Fig. 1; an interventional device, such as the catheter 150 illustrated in Fig. 1, for example; display, such as the display 230 illustrated in Fig. 1, for displaying the image representation 180 of the vascular region, other outputs generated by the one or more processors 110, the one or more projected angiographic images 130, the live sequence of projected fluoroscopic images 140 of the vascular region, and so forth; an injector (not illustrated in Fig. 1) for injecting a contrast agent into the vasculature of a subject; a patient bed 240; and a user input device (not illustrated in Fig. 1) configured to receive user input in relation to the operations performed by the one or more processors 110, such as a keyboard, a mouse, a touchscreen, and so forth.

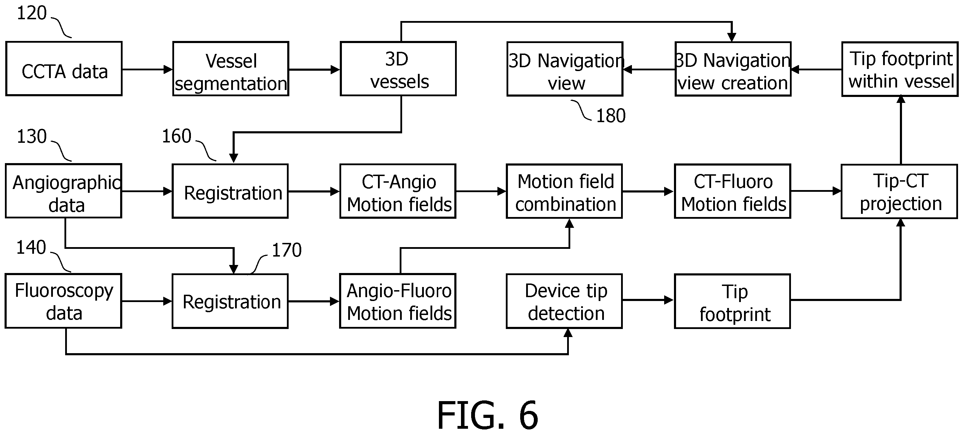

Some elements of the method that are performed by the processors 110 of the above system 100 are also illustrated in Fig. 6, which is a schematic diagram illustrating a second example of various elements of a method of providing an image representation of a vascular region, in accordance with some aspects of the present disclosure. In the example illustrated in Fig. 6, the first image data is CCTA data, i.e. data corresponding to a coronary computed tomography angiographic image 120. In this example, the second image data is angiographic data 130, and the third image data is fluoroscopy data.

In another example, the system 100 described above, is included within a projection X-ray imaging system. In this example, a projection X-ray imaging system comprises a system 100 for providing an image representation of a vascular region. The system comprises one or more processors 110 configured to:
receive S110 first image data representing a volumetric angiographic image 120 of the vascular region;
receive S120 second image data representing one or more projected angiographic images 130 of the vascular region;
receive S130, from the projection X-ray imaging system, third image data representing a live sequence of projected fluoroscopic images 140 of the vascular region, the live sequence of projected fluoroscopic images including at least a portion of an interventional device 150;
calculate S140 three-dimensional positions of the at least a portion of the interventional device 150 in the volumetric angiographic image 120 corresponding to two-dimensional positions of the at least a portion of the interventional device 150 in the live sequence of projected fluoroscopic images 140, based on a first registration operation 160 wherein the volumetric angiographic image 120 is registered to the one or more projected angiographic images 130, and a second registration operation 170 wherein the live sequence of projected fluoroscopic images 140 is registered to the one or more projected angiographic images 130; and
output S150 an image representation 180 of the vascular region, wherein the image representation 180 is determined based on the calculated three-dimensional positions of the at least a portion of the interventional device 150 in the volumetric angiographic image 120.

In another example, a computer-implemented method of providing an image representation of a vascular region, is provided. The computer-implemented method comprises:
receiving S110 first image data representing a volumetric angiographic image 120 of the vascular region;
receiving S120 second image data representing one or more projected angiographic images 130 of the vascular region;
receiving S130 third image data representing a live sequence of projected fluoroscopic images 140 of the vascular region, the live sequence of projected fluoroscopic images including at least a portion of an interventional device 150;
calculating S140 three-dimensional positions of the at least a portion of the interventional device 150 in the volumetric angiographic image 120 corresponding to two-dimensional positions of the at least a portion of the interventional device 150 in the live sequence of projected fluoroscopic images 140, based on a first registration operation 160 wherein the volumetric angiographic image 120 is registered to the one or more projected angiographic images 130, and a second registration operation 170 wherein the live sequence of projected fluoroscopic images 140 is registered to the one or more projected angiographic images 130; and
outputting S150 an image representation 180 of the vascular region, wherein the image representation 180 is determined based on the calculated three-dimensional positions of the at least a portion of the interventional device 150 in the volumetric angiographic image 120.

In another example, a computer program product, is provided. The computer program product comprises instructions which when executed by one or more processors 110, cause the one or more processors to carry out a method of providing an image representation of a vascular region. The method comprises:
receiving S110 first image data representing a volumetric angiographic image 120 of the vascular region;
receiving S120 second image data representing one or more projected angiographic images 130 of the vascular region;
receiving S130 third image data representing a live sequence of projected fluoroscopic images 140 of the vascular region, the live sequence of projected fluoroscopic images including at least a portion of an interventional device 150;
calculating S140 three-dimensional positions of the at least a portion of the interventional device 150 in the volumetric angiographic image 120 corresponding to two-dimensional positions of the at least a portion of the interventional device 150 in the live sequence of projected fluoroscopic images 140, based on a first registration operation 160 wherein the volumetric angiographic image 120 is registered to the one or more projected angiographic images 130, and a second registration operation 170 wherein the live sequence of projected fluoroscopic images 140 is registered to the one or more projected angiographic images 130; and
outputting S150 an image representation 180 of the vascular region, wherein the image representation 180 is determined based on the calculated three-dimensional positions of the at least a portion of the interventional device 150 in the volumetric angiographic image 120.

The above examples are to be understood as illustrative of the present disclosure, and not restrictive. Further examples are also contemplated. For instance, the examples described in relation to the system 100, may also be provided by the computer-implemented method, or by the computer program product, or by the computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing an image representation of a vascular region, the system comprising one or more processors (110) configured to:
receive (S110) first image data representing a volumetric angiographic image (120) of the vascular region;
receive (S120) second image data representing one or more projected angiographic images (130) of the vascular region;
receive (S130) third image data representing a live sequence of projected fluoroscopic images (140) of the vascular region, the live sequence of projected fluoroscopic images including at least a portion of an interventional device (150);
calculate (S140) three-dimensional positions of the at least a portion of the interventional device (150) in the volumetric angiographic image (120) corresponding to two-dimensional positions of the at least a portion of the interventional device (150) in the live sequence of projected fluoroscopic images (140), based on a first registration operation (160) wherein the volumetric angiographic image (120) is registered to the one or more projected angiographic images (130), and a second registration operation (170) wherein the live sequence of projected fluoroscopic images (140) is registered to the one or more projected angiographic images (130); and
output (S150) an image representation (180) of the vascular region, wherein the image representation (180) is determined based on the calculated three-dimensional positions of the at least a portion of the interventional device (150) in the volumetric angiographic image (120).

2. The system according to claim 1, wherein the first registration operation (160) and the second registration operation (170) define a transform comprising a back-projection of the two-dimensional positions of the at least a portion of the interventional device (150) lying in a plane in the live sequence of projected fluoroscopic images (140), along a depth axis (190) onto the corresponding three-dimensional positions of the at least a portion of the interventional device (150) in the volumetric angiographic image (120), and wherein each three-dimensional position comprises a back-projection depth (D) along the depth axis (190), the back-projection depth (D) being within a vessel in the vascular region, and the depth axis being defined normally with respect to the plane of the live sequence of projected fluoroscopic images (140).

3. The system according to claim 2, wherein for two-dimensional positions of the at least a portion of the interventional device (150) in the live sequence of projected fluoroscopic images (140) which when back-projected along the depth axis (190) intersect with multiple vessels (210₁, 210₂) in the volumetric angiographic image (120), the one or more processors (110) are configured to calculate (S140) the three-dimensional position of the at least a portion of the interventional device (150) in the volumetric angiographic image (120) by back-projecting the two-dimensional position of the at least a portion of the interventional device (150) into a selected one of the intersected vessels (210₁, 210₂) to define the back-projection depth; and
wherein the selected intersected vessel (210₁, 210₂) is determined based on one or more of:
a comparison between an orientation of the at least a portion of the interventional device (150) in the live sequence of projected fluoroscopic images (140), and an orientation of the intersected vessel (210₁, 210₂) in the volumetric angiographic image (120);
a comparison between a measurement of a dimension of a feature of the vessel (210₁, 210₂) in the one or more projected angiographic images (130), and a corresponding measurement of the dimension of the feature in the intersected vessel (210₁, 210₂) in the volumetric angiographic image (120); and
a comparison between a position of an anatomical landmark in the vessel (210₁, 210₂) in the one or more projected angiographic images (130), and a corresponding position of the anatomical landmark in the intersected vessel (210₁, 210₂) in the volumetric angiographic image (120).

4. The system according to any previous claim, wherein the one or more processors (110) are configured to calculate (S140) the three-dimensional positions of the at least a portion of the interventional device (150) in the volumetric angiographic image (120) such that the two-dimensional positions of the at least a portion of the interventional device (150) in consecutive images in the live sequence of projected fluoroscopic images (140) are mapped to corresponding three-dimensional positions in the same vessel (210_{1..i}) in the volumetric angiographic image (120).

5. The system according to any previous claim, wherein the vascular region comprises a plurality of vessels (210_{1..i}), and wherein the one or more processors (110) are further configured to label the vessels (210_{1..i}) in the volumetric angiographic image (120), and in the one or more projected angiographic images (130); and
wherein the one or more processors (110) are configured to calculate (S140) the three-dimensional positions of the at least a portion of the interventional device (150) in the volumetric angiographic image (120) by:
mapping the two-dimensional positions of the at least a portion of the interventional device (150) in the live sequence of projected fluoroscopic images (140), to the corresponding three-dimensional positions of the at least a portion of the interventional device (150) in the volumetric angiographic image (120), based on the first registration operation (160), and the second registration operation (170), and via the one or more projected angiographic images (130), such that the two-dimensional positions of the at least a portion of the interventional device (150) in the vessels (210_{1..i}) in the live sequence of projected fluoroscopic images (140), are mapped to the corresponding three-dimensional positions of the at least a portion of the interventional device (150) in the same vessel (210_{1..i}) in both the one or more projected angiographic images (130) and in the volumetric angiographic image (120).

6. The system according to any previous claim, wherein the vascular region comprises a plurality of vessels (210_{1..i}), and wherein the one or more processors (110) are further configured to prune the vessels (210_{1..i}) in the volumetric angiographic image (120) such that the vessels in the volumetric angiographic image correspond to the vessels in the one or more projected angiographic images (130).

7. The system according to any previous claim, wherein the vascular region comprises a plurality of vessels (210_{1..i}), and wherein the one or more processors (110) are configured to calculate (S140) the three-dimensional positions of the at least a portion of the interventional device (150) in the volumetric angiographic image (120) by:
fitting a three-dimensional model of the interventional device (150) to the at least a portion of the interventional device in the live sequence of projected fluoroscopic images (140); and
constraining the three-dimensional positions of the at least a portion of the interventional device (150) in the volumetric angiographic image (120), using the fitted three-dimensional model, such that the three-dimensional positions of the at least a portion of the interventional device (150) lie within one of the vessels (210_{1..i}) in the volumetric angiographic image (120).

8. The system according to any previous claim, wherein the one or more processors (110) are configured to calculate (S140) the three-dimensional positions of the at least a portion of the interventional device (150) in the volumetric angiographic image (120) by:
mapping the positions of the at least a portion of the interventional device (150) from the live sequence of projected fluoroscopic images (140) to corresponding nearest positions along a centerline of a vessel in the volumetric angiographic image (120), or to corresponding nearest positions in the vessel lying on a surface oriented normally with respect to a depth dimension of the vessel and passing half-way through the vessel along the depth dimension.

9. The system according to any previous claim, wherein the one or more processors (110) are further configured to:
evaluate an accuracy parameter for the image representation (180) based on a deviation between the two-dimensional positions of the at least a portion of the interventional device (150) in the live sequence of projected fluoroscopic images (140), and the corresponding three-dimensional positions of the at least a portion of the interventional device (150) in the volumetric angiographic image (120); and
output the value of the accuracy parameter.

10. The system according to any previous claim, wherein the first registration operation (160) comprises a rigid registration and/or wherein the second registration operation (170) comprises an elastic registration.

11. The system according to any previous claim, wherein the volumetric angiographic image (120) of the vascular region, and the one or more projected angiographic images (130) of the vascular region, each represent a distribution of a contrast agent in the vascular region.

12. The system according to any previous claim, wherein the live sequence of projected fluoroscopic images (140) is acquired in the absence of a contrast agent in the vascular region.

13. The system according to any previous claim, wherein the second angiographic image data represents a sequence of projected angiographic images (130) of the vascular region; and
wherein the sequence of projected angiographic images (130) is acquired prior to the live sequence of projected fluoroscopic images (140).

14. The system according to any previous claim, wherein the image representation (180) comprises:
the at least a portion of the interventional device (150) in the calculated three-dimensional positions in the volumetric angiographic image (120); and/or
a perspective view of the vascular region from the calculated three-dimensional positions of the at least a portion of the interventional device (150) in the volumetric angiographic image (120).

15. A computer-implemented method of providing an image representation of a vascular region, the method comprising:
receiving (S110) first image data representing a volumetric angiographic image (120) of the vascular region;
receiving (S120) second image data representing one or more projected angiographic images (130) of the vascular region;
receiving (S130) third image data representing a live sequence of projected fluoroscopic images (140) of the vascular region, the live sequence of projected fluoroscopic images including at least a portion of an interventional device (150);
calculating (S140) three-dimensional positions of the at least a portion of the interventional device (150) in the volumetric angiographic image (120) corresponding to two-dimensional positions of the at least a portion of the interventional device (150) in the live sequence of projected fluoroscopic images (140), based on a first registration operation (160) wherein the volumetric angiographic image (120) is registered to the one or more projected angiographic images (130), and a second registration operation (170) wherein the live sequence of projected fluoroscopic images (140) is registered to the one or more projected angiographic images (130); and
outputting (S150) an image representation (180) of the vascular region, wherein the image representation (180) is determined based on the calculated three-dimensional positions of the at least a portion of the interventional device (150) in the volumetric angiographic image (120).
